(19) Europäisches Patentamt / European Patent Office / Office européen des brevets

(11) **EP 4 194 018 A1**

(12) **EUROPEAN PATENT APPLICATION**
published in accordance with Art. 153(4) EPC

(43) Date of publication:
**14.06.2023 Bulletin 2023/24**

(21) Application number: **21852270.4**

(22) Date of filing: **04.08.2021**

(51) International Patent Classification (IPC):
**A61L 26/00** *(2006.01)*     **A61K 47/32** *(2006.01)*

(52) Cooperative Patent Classification (CPC):
**A61K 47/32; A61L 26/00**

(86) International application number:
**PCT/JP2021/028942**

(87) International publication number:
**WO 2022/030540 (10.02.2022 Gazette 2022/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.08.2020   PCT/JP2020/030050**

(71) Applicant: **Maruho Co., Ltd.
Osaka 531-0071 (JP)**

(72) Inventors:
- **NAKAMURA Yuki**
  **Osaka-shi, Osaka 531-0071 (JP)**
- **ISHIGAME Takayoshi**
  **Osaka-shi, Osaka 531-0071 (JP)**
- **YAMAKOSHI Wataru**
  **Osaka-shi, Osaka 531-0071 (JP)**

(74) Representative: **Pfenning, Meinig & Partner mbB
Patent- und Rechtsanwälte
Theresienhöhe 11a
80339 München (DE)**

(54) **SKIN COMPOSITION**

(57)     The present invention relates to a film-forming skin composition containing (a1) an ethyl acrylate-methyl methacrylate-trimethylammonium ethyl methacrylate chloride copolymer, and (a2) an ethyl acrylate-methyl methacrylate copolymer. The film formed from the composition of the present invention has high water resistance and/or high friction resistance. Meanwhile, this film can be easily washed off from the skin.

EP 4 194 018 A1

**Description**

TECHNICAL FIELD

The present invention relates to a film-forming composition, specifically, a film-forming skin protectant and a film-forming skin topical agent.

BACKGROUND ART

A liquid adhesive bandage that forms a thin film is considered to be effective for symptoms in which the skin barrier function is deteriorated (hangnail, rubbing, cracking, etc.), and is commercially available for the purpose of protecting skin from kitchen work or the like. However, while the film is desired to have high water resistance and high friction resistance, the film is desired to be easily washed off from the skin (high washed-off property). This is because when it is difficult to wash off the film, there are problems that when a side effect occurs, the film cannot be quickly removed from the skin (as a result, administration cannot be promptly discontinued), and an old film tends to remain, so that the skin permeation amount of a medicinal ingredient contained in a new film formed on the old film is reduced.
Patent Documents 1 and 2 disclose a topical agent for fingers that forms a soft film when applied to entire fingers, is excellent in quick-drying properties and adhesion to the skin, and can be washed away with an alkaline solution after use. Patent Document 3 discloses a film skin protectant that can form a thin and transparent film, has good usability, is excellent in water resistance, and is easy to wash with alkaline soap. However, any of the compositions disclosed in Patent Documents 1 to 3 has a problem that the film cannot be easily washed away with water (or hot water) after use.

PRIOR ART DOCUMENT

PATENT DOCUMENTS

Patent Document 1: JP-A-2011-126796
Patent Document 2: JP-A-2011-126797
Patent Document 3: JP-A-H05-32535

SUMMARY OF THE INVENTION

PROBLEMS TO BE SOLVED BY THE INVENTION

An object of the present invention is to provide a film-forming skin composition, specifically, a film-forming skin protectant and a film-forming skin topical agent, which can form a film having high water resistance and/or high friction resistance, while having excellent washed-off property (easily washed off from the skin).

MEANS FOR SOLVING THE PROBLEMS

As a result of repeated studies to solve the above problems, the present inventors have found that the above problems can be solved by combining specific polymers, and have completed the present invention.
That is, the present invention relates to the following [1] to [10].

[1] A film-forming skin composition containing the following two polymers:

(a1) an ethyl acrylate-methyl methacrylate-trimethylammonium ethyl methacrylate chloride copolymer; and
(a2) an ethyl acrylate-methyl methacrylate copolymer.

[2] The composition according to [1], containing propranolol or a pharmaceutically acceptable salt thereof as a medicinal ingredient.
[3] The composition according to [2], which is used for treatment of hemangioma.
[4] The composition according to [2] or [3], which is used for treatment of hemangioma in infants.
[5] The composition according to any one of [1] to [4], in which a weight ratio of the polymer (a1) to the polymer (a2) is in a range of 8 : 2 to 2 : 8.
[6] The composition according to any one of [1] to [5], further containing 36% by weight or more of water based on the total amount of the composition (provided that a propellant is excluded when the composition is an aerosol).

[7] The composition according to any one of [1] to [6], further containing a surfactant.

[8] The composition according to [7], in which the surfactant is a nonionic surfactant and/or an amphoteric surfactant.

[9] The composition according to any one of [1] to [8], further containing one or more plasticizers selected from the group consisting of an ester compound that is liquid at ordinary temperature, an aromatic alcohol that is liquid at ordinary temperature, a medium-polar solid ester compound, and a terpene.

[10] The composition according to any one of [1] to [9], wherein the composition is substantially free of a lower monohydric alcohol.

EFFECT OF THE INVENTION

A film formed from the composition of the present invention has high water resistance and/or high friction resistance, and even has excellent washed-off property. The composition of the present invention can be used as a film-forming skin protectant and a film-forming skin topical agent.

MODE FOR CARRYING OUT THE INVENTION

The composition of the present invention is characterized in that it contains a combination of two specific polymers. The composition of the present invention can be specifically used as a film-forming skin protectant and a film-forming skin topical agent.

The film-forming skin protectant (not containing a medicinal ingredient) can form a thin film by being sprayed or applied onto skin to effectively protect the skin, and can be used for prevention or treatment of skin diseases such as hand eczema typified by housewife eczema, asteatosis, and atopic dermatitis.

In addition, a film-forming skin topical agent (including a medicinal ingredient) forms a film by being sprayed or applied onto skin, and the medicinal ingredient can be effectively delivered to the skin. In addition, the same effect can be expected when the composition is applied to a nail or a skin around the nail or a mucous membrane.

The two polymers used in the present invention are:

(a1) an ethyl acrylate-methyl methacrylate-trimethylammonium ethyl methacrylate chloride copolymer; and

(a2) an ethyl acrylate-methyl methacrylate copolymer.

The weight ratio of the polymer (a1) to the polymer (a2) is preferably 8 : 2 to 2 : 8, more preferably 8 : 2 to 5 : 5, particularly preferably 8 : 2 to 6 : 4, and further preferably 8 : 2 to 7 : 3.

The average molecular weight (Mw) of the ethyl acrylate-methyl methacrylate-trimethylammonium ethyl methacrylate chloride copolymer (a1) is preferably in the range of 10,000 to 100,000, and more preferably in the range of 20,000 to 50,000. Examples of commercially available products of the polymer (a1) include EUDRAGIT[(registered trademark)] RS type and EUDRAGIT RL type (both available from Evonik Japan Co., Ltd.).

EUDRAGIT RS type is an acrylic acid-based polymer with a composition ratio of components of 1 : 2 : 0.1. EUDRAGIT RS type has an average molecular weight (Mw) of about 32,000 and is insoluble in water regardless of pH. EUDRAGIT RS type is available, for example, as EUDRAGIT RS100, EUDRAGIT RSPO, and EUDRAGIT RS30D (an aqueous suspension containing 30% by weight of an ethyl acrylate-methyl methacrylate-trimethylammonium ethyl methacrylate chloride copolymer (composition ratio 1 : 2 : 0.1)).

EUDRAGIT RL type is an acrylic acid-based polymer with a composition ratio of components of 1 : 2 : 0. 2. EUDRAGIT RL type has an average molecular weight (Mw) of about 32,000 and is insoluble in water regardless of pH. EUDRAGIT RL type is available as EUDRAGIT RL100, EUDRAGIT RLPO, and EUDRAGIT RL30D (an aqueous suspension containing 30% by weight of ethyl acrylate-methyl methacrylate-trimethylammonium ethyl methacrylate chloride copolymer (composition ratio 1 : 2 : 0.2)).

The average molecular weight (Mw) of the ethyl acrylate-methyl methacrylate copolymer (a2) is preferably in the range of 100,000 to 1,500,000, and more preferably in the range of 500,000 to 1,000,000. Examples of commercially available products of the polymer (a2) include EUDRAGIT NE type (Evonik Japan Co., Ltd.). EUDRAGIT NE type is an acrylic acid-based polymer with a composition ratio of components of 2 : 1. EUDRAGIT NE type has an average molecular weight (Mw) of about 750,000 and is insoluble in water regardless of pH. EUDRAGIT NE type is available, for example, as EUDRAGIT NE30D (an aqueous suspension containing 30% by weight of ethyl acrylate-methyl methacrylate copolymer (composition ratio 2 : 1))

The content (total content) of the polymers based on the total amount of the composition of the present invention (provided that a propellant is excluded when the composition is an aerosol) is appropriately 2% by weight or more, 3% by weight or more, or 4% by weight or more. For example, the content is preferably 6 to 35% by weight, more preferably 10 to 30% by weight, and particularly preferably 12 to 20% by weight. Since the composition of the present invention is excellent in water resistance, it may not particularly contain a silicone-based polymer.

Film-forming compositions often contain a lower monohydric alcohol such as ethanol or isopropanol in order to enhance quick-drying properties, but the composition of the present invention is excellent in quick-drying properties and can quickly form a film even without containing a lower monohydric alcohol (monohydric alcohol having 1 to 3 carbon atoms, for example, ethanol, isopropanol, or the like). The composition of the present invention may contain a lower monohydric alcohol, and the content thereof is preferably 10% by weight or less, more preferably 7% by weight or less, particularly preferably 5% by weight or less, and further preferably 3% by weight or less based on the total amount of the composition.

A preferred embodiment of the present invention is a composition that is substantially free of a lower monohydric alcohol. Substantially free means that a lower monohydric alcohol is not intentionally added in the production process. That is, the lower monohydric alcohol content in the composition substantially free of a lower monohydric alcohol is usually 0% by weight, and even if it is mixed very slightly, the content is less than 1% by weight (more preferably less than 0.5% by weight).

Usually, in film-forming compositions, a lower monohydric alcohol such as ethanol or isopropanol is used to dissolve a polymer as a film-forming component, in addition to the improvement in quick-drying properties. However, when used for treatment of a skin disease with deteriorated barrier function such as hand eczema or atopic dermatitis or a disease in infants whose skin is thinner than that of adults, it is preferable not to contain a lower monohydric alcohol causing skin dryness or irritation. Since the present invention can provide a composition substantially free of a lower monohydric alcohol, it is possible to provide a composition that has high safety to the skin and is suitable for treating hand eczema and atopic dermatitis, and diseases in infants.

The composition of the present invention preferably contains at least one surfactant (emulsifier). By adding a surfactant to the composition, a residue of the film after washing can be reduced and/or the visibility of the film can be improved. By enhancing the visibility of the film, it is easy to confirm whether or not the film has been removed after washing. The surfactant is selected from the group consisting of nonionic surfactants, cationic surfactants, anionic surfactants, and amphoteric surfactants. Nonionic surfactants and amphoteric surfactants are more preferred, and nonionic surfactants are particularly preferred. The content of the surfactant based on the total amount of the composition (provided that a propellant is excluded when the composition is an aerosol) is preferably 0.1 to 5% by weight, more preferably 0.3 to 4% by weight, and particularly preferably 0.5 to 3% by weight. Examples of suitable compositions include compositions containing 0.8% by weight or more or 1% by weight or more of the surfactant. The surfactants may be used singly or in combination of two or more types thereof.

Examples of the nonionic surfactant include polyoxyethylene alkyl ethers such as polyoxyethylene oleyl ether, polyoxyethylene octyldodecyl ether, and polyoxyethylene lauryl ether; polyoxyethylene alkylphenol ether; polyoxyethylene hydrogenated castor oil; polyoxyl stearate; glycerin fatty acid esters such as glyceryl monostearate, self-emulsifying glyceryl monostearate, glyceryl monoisostearate, glyceryl palmitate, glyceryl myristate, glyceryl oleate, and glyceryl triisooctanoate; diglycerol fatty acid esters such as diglyceryl laurate, diglyceryl stearate, and diglyceryl oleate; polyglycerin fatty acid esters such as decaglyceryl monolaurate; polyoxyethylene glycerin fatty acid esters such as polyoxyethylene glyceryl monostearate; sorbitan fatty acid esters such as sorbitan monopalmitate, sorbitan monostearate, sorbitan monooleate, sorbitan coconut oil fatty acid, sorbitan tristearate, and sorbitan trioleate; polyethylene glycol fatty acid esters such as polyethylene glycol monolaurate, polyethylene glycol monostearate, and polyethylene glycol monooleate; polyoxyethylene sorbitan fatty acid esters such as polyoxyethylene sorbitan tristearate, and polyoxyethylene coconut oil fatty acid sorbitan, and the like. In particular, polyoxyethylene hydrogenated castor oil, polyoxyethylene sorbitan tristearate, polyoxyethylene lauryl ether, polyoxyethylene coconut oil fatty acid sorbitan, polyethylene glycol monooleate, decaglyceryl monolaurate, diglyceryl monooleate, and polyoxyethylene glyceryl monostearate are preferred.

Specific examples of the cationic surfactant include cetyltrimethylammonium chloride, lauryldimethylbenzylammonium chloride, tetrabutylammonium chloride, dioctadecyldimethylammonium chloride, and the like.

Examples of the anionic surfactant include sodium alkylbenzene sulfonate, sodium dodecyl sulfate, coconut alcohol sodium ethoxy sulfate, sodium α-olefin sulfonate, emulsified cetostearyl alcohol, sodium lauroyl sarcosine, potassium myristate, and the like.

Examples of the amphoteric surfactant include N-alkyl-N,N-dimethylammonium betaine, lauryldimethylaminoacetic acid betaine, coconut oil fatty acid amidopropyldimethylaminoacetic acid betaine, imidazoline-type amphoteric surfactants, and the like.

The composition of the present invention may contain a plasticizer. Examples of the plasticizer include an ester compound that is liquid at ordinary temperature (25°C; the same applies hereinafter), an aromatic alcohol that is liquid at ordinary temperature, a medium-polar solid ester compound, and a terpene. These may be used singly or in combination of two or more types thereof. By using such a plasticizer, the film formation time is shortened (that is, quick-drying properties are improved).

Preferred examples of the ester compound that is liquid at ordinary temperature include triethyl citrate, triacetin, dibutyl phthalate, diethyl sebacate, diisopropyl sebacate, diisopropyl adipate, medium-chain triglycerides (for example, glyceryl triisooctanoate, tri(caprylic/capric acid) glyceryl, and the like).

Fatty acid esters which are selected from diethyl sebacate, diisopropyl sebacate, diisopropyl adipate and medium-chain triglycerides and are liquid at ordinary temperature are more preferable, and among them, diethyl sebacate and diisopropyl adipate are further preferable, and diisopropyl adipate is particularly preferable.

Preferred examples of the aromatic alcohol that is liquid at ordinary temperature include ethylene glycol salicylate and phenoxyethanol, and phenoxyethanol is particularly preferred.

Examples of the medium-polarity solid ester compound include ester compounds that are solid at ordinary temperature, such as phospholipid (for example, lecithin, especially hydrogenated lecithin) and parahydroxybenzoic acid ester. In the present specification, the medium polarity means that $\alpha$ value according to an organic conceptual diagram is 35° to 55°. The organic conceptual diagram was proposed by Atsushi Fujita, and details thereof are described in "Pharmaceutical Bulletin", 1954, vol. 2, 2, pp. 163 to 173; "Chemistry region", 1957, vol. 11, 10, pp. 719 to 725; "Fragrance journal", 1981, vol. 50, pp. 79 to 82, and the like. That is, a source of all the organic compounds is methane ($CH_4$), and all the other compounds are regarded as derivatives of methane, and certain numerical values are set for a carbon number, substituent, modification part and ring thereof and the like, and the scores are added to determine an organic value and an inorganic value. An angle of inclination when the values are plotted on a diagram in which the organic value is taken on an X-axis and the inorganic value is taken on a Y-axis is the $\alpha$ value.

Examples of combined use of an ester compound that is liquid at ordinary temperature include a combination of a medium-chain triglyceride (for example, glyceryl triisooctanoate, tri(caprylic/capric acid) glyceryl, and the like), and triethyl citrate, triacetin, dibutyl phthalate, diethyl sebacate, diisopropyl sebacate or diisopropyl adipate.

Examples of combined use of an ester compound that is liquid at ordinary temperature and an aromatic alcohol that is liquid at ordinary temperature include a combination of a medium-chain triglyceride (for example, glyceryl triisooctanoate, tri(caprylic/capric acid) glyceryl, and the like), and ethylene glycol salicylate or phenoxyethanol.

Preferred examples of the terpenes include limonene and menthol.

The content of the plasticizer based on the total amount of the composition of the present invention (provided that a propellant is excluded when the composition is an aerosol) may be 0.5% by weight or more or 1% by weight or more, for example, is preferably 1.3 to 35% by weight, more preferably 1.5 to 20% by weight, particularly preferably 1.7 to 12% by weight, and further preferably 2 to 8% by weight. When the amount of the plasticizer is less than 1.3% by weight, film formability and water resistance are deteriorated, and when the amount is more than 35% by weight, the film tends to be sticky, which is not preferable.

The composition of the present invention can contain water. The water used in the present invention is particularly preferably purified water. The content of water based on the total amount of the composition of the present invention (provided that a propellant is excluded when the composition is an aerosol) is preferably 36% by weight or more, and more preferably 50% by weight or more. More specifically, the content is preferably 36 to 92% by weight, more preferably 43 to 89% by weight, particularly preferably 50 to 87% by weight, and further preferably 64 to 85% by weight. The composition containing a large amount (36% by weight or more) of water has a fresh feeling of use. EUDRAGIT RS30D, RL30D, and NE30D are all provided as aqueous suspensions containing 30% by weight of an acrylic polymer. Water contained in the aqueous suspensions (corresponding to 70% by weight of the aqueous suspensions) also corresponds to the water used in the present invention.

The composition of the present invention can contain, for example, one or more substances selected from light anhydrous silicic acid, titanium oxide, talc, and calcium carbonate in order to suppress gloss of the film. The content of the substance based on the total amount of the composition of the present invention (provided that a propellant is excluded when the composition is an aerosol) is preferably 0.8 to 3% by weight, and particularly preferably 1 to 3% by weight.

The composition of the present invention can contain a pH adjusting agent in addition to the above components. Examples of the pH adjusting agent include phosphate, citrate, hydroxide, hydrochloric acid, and the like. The pH adjusting agent may be used singly or in combination of two or more types thereof. The content of the pH adjusting agent based on the total amount of the composition of the present invention (provided that a propellant is excluded when the composition is an aerosol) is preferably 0.01 to 1% by weight, and particularly preferably 0.1 to 0.5% by weight.

In particular, when the pH of the composition is 4 or more, the water resistance of the film is improved, which is preferable. A preferred pH range is 5 to 9 and more preferably 6 to 8.

The composition of the present invention can further contain additives such as thickeners (for example, hydrophobically modified hydoroxypropyl methylcellulose [another name: hydroxypropyl methylcellulose stearoxy ether], gelatin, polyoxyethylene glycol, and the like), preservatives (for example, paraoxybenzoic acid esters, benzalkonium chloride, benzethonium chloride, phenoxyethanol, and the like), stabilizers (for example, dibutylhydroxytoluene, sodium citrate, citric acid, ascorbic acid, mannitol, sorbic acid, sorbitol, sodium edetate, cyclodextrins, and the like). The content of each of the additives based on the total amount (provided that a propellant is excluded when the composition is an aerosol) of the composition of the present invention is preferably 0.01 to 2% by weight, and more preferably 0.05 to 1% by weight, and the total content of each of the additives is preferably 5% by weight or less, more preferably 4% by weight or less, and particularly preferably 3% by weight or less.

The composition of the present invention can contain, for example, a medicinal ingredient effective for treatment of various skin diseases including chronic skin diseases represented by atopic dermatitis.

Examples of the medicinal ingredient include, but are not limited to, the following ingredients.

- Steroidal anti-inflammatory drugs such as hydrocortisone, dexamethasone, clobetasol 17-propionate, dexamethasone 17-valerate, fluocinonide, halcinonide, amcinonide, difluprednate, and betamethasone butyrate propionate;
- Non-steroidal anti-inflammatory drugs such as indomethacin, ketoprofen, flurbiprofen, felbinac, piroxicam, ibuprofen piconol, bendazac, butyl flufenamate, and bufexamac;
- Anti-fungal drugs such as lanoconazole, tolnaftate, clotrimazole, bifonazole, miconazole nitrate, econazole nitrate, ketoconazole nitrate, omoconazole nitrate, oxiconazole nitrate, exalamide, tricyclate, and siccanin;
- Antimicrobials such as ozenoxacin and clindamycin;
- Anti-allergic drugs such as ketotifen, azelastine and salts thereof, chlorpheniramine maleate, oxatomide, tranilast, and sodium cromoglicate;
- Local anesthetics such as lidocaine and propitocaine;
- Antiviral drugs such as aciclovir, valacyclovir, famciclovir, amenamevir, vidarabine, and salts thereof;
- Humectants such as heparinoids, hyaluronic acid, and urea;
- Fibroblast growth factor and wound therapeutic agents such as bucladesine sodium;
- Therapeutic agents for acne, such as benzoyl peroxide and adapalene;
- Therapeutic agents for keratosis including psoriasis vulgaris such as maxacalcitol;
- Therapeutic agents for arrhythmia, hypertension, myocardial infarction, angina pectoris, migraine and hemangioma such as β-blockers;
- Therapeutic agents for warts vulgaris such as salicylic acid, mono-trichloroacetic acid, glutaraldehyde, phenol, tacalcitol, calcipotriol, maxacalcitol, calcitriol, alfacalcidol, eldecalcitol, bleomycin, 5-FU, podophyllin, retinoid, coix seed extract, imiquimod, cimetidine, and interferon;
- Therapeutic agents for postherpetic neuralgia such as aciclovir, valacyclovir, famciclovir, amenamevir, vidarabine, pregabalin, gabapentin, duloxetine, amitriptyline, nortriptyline, imipramine, an extract liquid from inflammatory rabbit skin inoculated by vaccinia virus, tramadol, tramadol/acetaminophen, fentanyl, oxycodone, buprenorphine, mexiletine, epalrestat, and morphine.

Examples of the β-blockers include acebutolol, betaxolol, carteolol, carvedilol, labetalol, oxprenolol, penbutolol, pindolol, and propranolol. The composition of the present invention containing such a β-blocker can be used as a therapeutic agent for arrhythmia, hypertension, migraine, hemangioma, and the like.

When using a β-blocker, the composition of the present invention can be used for treatment of hemangioma (in particular, hemangioma in infants) and can be used for treatment of vascular tumors (however, the present invention is not limited thereto), for example, selected from the group consisting of capillary hemangioma, epithelioid hemangioma, sinusoidal hemangioma, spindle cell hemangioma, tufted angioma, hemangioendothelioma (that is, Kaposi-form hemangioendothelioma), hemangioma in von Hippel-Lindau syndrome, fibroangioma and hemangiolipoma in Bourneville's disease, pyogenic granuloma, hemangiosarcoma, for example, Kaposi's sarcoma, proliferative arteriovenous malformation, and tumor-associated vascular proliferation. When the composition of the present invention is used for treatment of hemangioma, it is preferably used in the growth phase.

One preferred example of the composition of the present invention is a composition containing propranolol or a pharmaceutically acceptable salt thereof. Examples of the pharmaceutically acceptable salt include salts formed from inorganic acids such as hydrochloric acid, bromic acid, sulfuric acid, nitric acid, and phosphoric acid, or salts formed from organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluene-sulfonic acid, and salicylic acid. In particular, compositions containing propranolol hydrochloride are preferred. Preferred indications for the composition of the present invention containing propranolol or a pharmaceutically acceptable salt thereof (in particular, propranolol hydrochloride) include hemangiomas (for example, hemangioma in infants).

The composition of the present invention not only has high water resistance and/or high friction resistance, but also has excellent washed-off property. Thus, when a side effect due to the medicinal ingredient contained in the film occurs, the film is quickly removed from the skin, whereby the use of the medicinal ingredient can be interrupted. Therefore, the composition of the present invention is particularly suitable as a therapeutic agent for disease in infants for which high safety is required.

The composition of the present invention can be used in the form of, but not limited to, a liquid, a lotion, a gel, a cream, an aerosol, or the like.

The content of the medicinal ingredient based on the total amount of the composition of the present invention (provided that a propellant is excluded when the composition is an aerosol) is preferably 0.01 to 5% by weight, more preferably 0.1 to 1% by weight, and particularly preferably 0.1 to 0.5% by weight.

The composition of the present invention may contain other components other than the above-described polymers, medicinal ingredient, plasticizer, surfactant, light anhydrous silicic acid, titanium oxide, talc, calcium carbonate, pH adjuster, additive, and water, and the content of other components based on the total amount (provided that a propellant is excluded when the composition is an aerosol) of the composition of the present invention is preferably 5% by weight or less, more preferably 3% by weight or less, and particularly preferably 1% by weight or less in total.

The composition of the present invention may be an aerosol containing a propellant. Examples of the propellant include dimethyl ether (DME) or a mixture of DME and liquefied natural gas (LPG). More preferred examples of the propellant include DME.

The content of the propellant in the aerosol of the present invention is preferably 30 to 50 parts by weight, more preferably 35 to 50 parts by weight, and particularly preferably 40 to 46 parts by weight, based on 100 parts by weight of the composition excluding the propellant.

Application amount and application frequency of the composition of the present invention to the skin may be appropriately adjusted according to skin symptoms, concentration of drug in the composition, age of patient, and the like. Usually, one to several applications per day are appropriate.

Although preferred compound names of essential components and optional components used in the composition of the present invention have been described in the preceding paragraph, the composition of the present invention also includes compositions obtained by arbitrarily combining these components and compositions obtained by arbitrarily combining the concentration ranges of the respective components. In addition, the numerical ranges such as the concentration can be arbitrarily combined, and when a plurality of numerical ranges is described, the upper limit value or the lower limit value of each numerical range can also be arbitrarily combined.

Hereinafter, the present invention will be described in more detail with reference to examples, but the present invention is not limited to examples.

<Polymers Used>

(a1) An ethyl acrylate-methyl methacrylate-trimethylammonium ethyl methacrylate chloride copolymer (composition ratio of components of 1 : 2 : 0.1, average molecular weight (Mw): about 32,000, insoluble in water regardless of pH).
(a2) An ethyl acrylate-methyl methacrylate copolymer (composition ratio of components of 2 : 1, average molecular weight (Mw): about 750,000, insoluble in water regardless of pH)

<Plasticizers Used>

Diisopropyl adipate (DID), triethyl citrate, triacetin, diethyl sebacate, diisopropyl sebacate, medium-chain triglyceride, ethylene glycol salicylate, phenoxyethanol

<Surfactants Used>

Polyoxyethylene hydrogenated castor oil (NIKKOL HCO-60)
Polyoxyethylene (20) sorbitan tristearate (NIKKOL TS-30V, Polysorbate 65/PS65)
Polyoxyethylene lauryl ether (4.2 E.O) (NIKKOL BL-4.2)
Polyoxyethylene coconut oil fatty acid sorbitan (20 E.O) (NIKKOL TL-10)
Polyethylene glycol monooleate (10 E.O.) (NIKKOL MYO-10V)
Decaglyceryl monolaurate (NIKKOL Decaglyn 1-L)
Diglyceryl monooleate (NIKKOL DGMO-90V)
Polyoxyethylene glyceryl monostearate (PEG-5) (NIKKOL TMGS-5V)
Polyoxyethylene glyceryl monostearate (PEG-15) (NIKKOL TMGS-15V)
Sodium lauroyl sarcosine (NIKKOL Sarcosinate LN)
Potassium myristate (NONSOUL MK-1)
Lauryl dimethylaminoacetate betaine (NIKKOLAM-301)
Coconut oil fatty acid amidopropyl dimethylaminoacetic acid betaine (NIKKOL AM-3130N)

<Thickener Used>

Hydroxypropyl methylcellulose stearoxy ether (hydrophobic HPMC)

<Medicinal Ingredient Used>

Propranolol hydrochloride

<Preparation Method>

Each polymer and purified water were weighed, and optionally plasticizer/surfactant/thickener/medicinal ingredient were added thereto, and the mixture was stirred until homogeneous. The composition of each formulation example (Formulation Examples 1 to 58) is as shown in Tables 2 and 4 to 11 (the numerical value of each component in the tables indicates % by weight). The contents of the polymer and the surfactant shown in the tables are solid contents. When an aqueous suspension (aqueous dispersion) or an aqueous solution comprising the polymer and/or the surfactant was used, the content of purified water shown in the tables is the total amount of water contained in the aqueous suspension or aqueous solution and added purified water.

[Example 1]

15 µL of the formulation was spread over the back of the hand of each panelist to a size of about 25 mm in diameter to form a film, and the visibility of the film was evaluated.
After the film was completely dried, the hand was immersed in hot water at 37°C for 30 seconds, the hand was removed from the hot water, the film was rubbed for 2 minutes, and the following two items of washed-off property were evaluated and scored (14 panelists for both washed-off property and visibility). The total score shown in Table 2 is a sum of values obtained by multiplying the numerical value of each score by the number of persons determined for the score.

[Table 1]

| Item | | Score | | | |
|---|---|---|---|---|---|
| | | 1 | 2 | 3 | 4 |
| Washed-off property | Washing time | 1 min and 30 sec or more | 1 min to 1 min and 30 sec | 30 sec to 1 min | 30 sec or less |
| | Residue of film | 70% or more remains | 30% to 70% remains | 30% or less remains | No residue |
| Visibility | | Hardly visible | Little hard to see | Somewhat easy to see | Easy to see |

[Table 2]

| | Formulation Example 1 | Formulation Example 2 | Formulation Example 3 | Formulation Example 4 |
|---|---|---|---|---|
| Polymer (a1) | 14.4 | 7.2 | 7.2 | 7.2 |
| Polymer (a2) | - | 7.2 | 7.2 | 7.2 |
| DID | 5 | 5 | 5 | 5 |
| HCO-60 | 2 | - | 1 | 2 |
| Purified water | 78.6 | 80.6 | 79.6 | 78.6 |
| Total amount | 100 | 100 | 100 | 100 |
| Evaluation item | | | | |
| Total score of washing time | 22 | 35 | 33 | 34 |

(continued)

| Evaluation item | | | | |
|---|---|---|---|---|
| Total score of residue of film | 35 | 46 | 49 | 51 |
| Total score of visibility | 35 | 20 | 34 | 36 |

As shown in Table 2, as compared with Formulation Example 1 in which only the polymer (a1) was used as the polymer, Formulation Examples 2 to 4 in which the polymers (a1) and (a2) were mixed resulted in a shorter washing time and less residue. In addition, when Formulation Examples 2 to 4 were compared, the amount of residue was smaller when the surfactant (HCO-60) was added. In particular, in Formulation Example 4 in which the concentration of the surfactant was high, 11 of 14 panelists evaluated that there was no residue (score 4). As for the visibility, it was confirmed that the visibility was increased depending on the concentration of the surfactant. On the other hand, the films formed on glass slides using Formulation Examples 2 to 4 were not peeled off even when they were soaked in water for 10 minutes and thus showed high water resistance. Therefore, it was confirmed that a film having both high water resistance and high washed-off property can be formed according to the present invention.

[Example 2]

Formulations (Formulation Examples 5 to 8) in which the mixing ratios of the polymers (a1) and (a2) were 9 : 1, 8 : 2, 7 : 3, and 6 : 4 were prepared, and the washed-off property thereof was compared with the washed-off property of Formulation Example 9 in which the mixing ratio of the polymers (a1) and (a2) was 5 : 5. Formulation Example 9 used as a comparative reference was obtained by adding a medicinal ingredient and a thickener to Formulation Example 4 which was confirmed to exhibit high washed-off property in Example 1, and thus Formulation Example 9 exhibits high washed-off property similar to Formulation Example 4.

<Method for Evaluating Washed-Off Property>

15 μL of the formulation was spread over the back of the hand of each panelist to a size of about 25 mm in diameter and dried for 5 minutes or more.
After the film dried, it was gently rubbed for 30 seconds using a body wiping sheet, and the washed-off property was evaluated and scored according to the following criteria (n = 4).

[Table 3]

| Item | Score | | | |
|---|---|---|---|---|
| | 1 | 2 | 3 | 4 |
| Comparison of washed-off property with Formulation Example 9 | Inferior | Little inferior | Equivalent | Better |

The composition and evaluation by each panelist of each formulation example are shown in the following table.

[Table 4]

| | Formulation Example 5 | Formulation Example 6 | Formulation Example 7 | Formulation Example 8 | Formulation Example 9 |
|---|---|---|---|---|---|
| Propranolol hydrochloride | 0.17 | 0.17 | 0.17 | 0.17 | 0.17 |
| Polymer (a1) | 13 | 11.5 | 10.1 | 8.6 | 7.2 |
| Polymer (a2) | 1.4 | 2.9 | 4.3 | 5.8 | 7.2 |
| Hydrophobic HPMC | 0.9 | 0.9 | 0.9 | 0.9 | 0.9 |
| DID | 5 | 5 | 5 | 5 | 5 |

(continued)

| | Formulation Example 5 | Formulation Example 6 | Formulation Example 7 | Formulation Example 8 | Formulation Example 9 |
|---|---|---|---|---|---|
| PS65 | 2 | 2 | 2 | 2 | 2 |
| Purified water | 77.53 | 77.53 | 77.53 | 77.53 | 77.53 |
| Total amount | 100 | 100 | 100 | 100 | 100 |
| Weight ratio of (a1) to (a2) | 9:1 | 8:2 | 7:3 | 6:4 | 5:5 |
| Score by each panelist | | | | | |
| Panelist 1 | 3 | 3 | 3 | 3 | - |
| Panelist 2 | 2 | 2 | 3 | 3 | - |
| Panelist 3 | 2 | 3 | 3 | 3 | - |
| Panelist 4 | 2 | 3 | 3 | 3 | - |

As shown in Table 4, the washed-off property of the formulation (Formulation Example 5) in which the ratio of the polymers (a1) and (a2) was 9 : 1 was not as high as that of Formulation Example 9, but the required washed-off criteria were satisfied. For the formulation (Formulation Example 6) in which the ratio of the polymers (a1) and (a2) was 8 : 2, higher washed-off property than that of Formulation Example 5 was observed, and the formulation (Formulation Example 7) in which the ratio of the polymers (a1) and (a2) was 7 : 3 and the formulation (Formulation Example 8) in which the ratio of the polymers (a1) and (a2) was 6 : 4 showed washed-off property equivalent to that of Formulation Example 9 in which the ratio of the polymers (a1) and (a2) was 5 : 5.

[Example 3]

A formulation containing only the polymer (a1) or a formulation containing the polymer (a1) and the polymer (a2) at a ratio of 7 : 3 or 5 : 5 were prepared, and friction resistance was evaluated according to the following criteria.

<Evaluation of Friction Resistance>

Mass (A) of a glass slide is measured.

100 $\mu$L of the formulation is uniformly applied to the entire surface of the glass slide using a spatula and dried in a mini-jet oven set at 30°C for 5 minutes. Thereafter, the glass slide is taken out from the mini-jet oven, and its mass (B) is measured. Next, a dry paper towel is placed on the formulation application surface of the glass slide, a weight of 100 g is placed thereon, the glass slide is wiped 20 times so that a force is uniformly applied to the entire glass slide, and then mass (C) of the glass slide is measured. The formulation residual ratio after wiping is calculated from each mass by the following formula. The test is repeated three times, and the average value and standard deviation of the formulation residual ratio are determined.

[Expression 1]

$$\text{Formulation residual ratio} = \frac{(C)-(A)}{(B)-(A)} \times 100$$

The composition and evaluation of the friction resistance of each formulation are shown in Table 5.

[Table 5]

| | Formulation Example 10 | Formulation Example 11 | Formulation Example 12 |
|---|---|---|---|
| Polymer (a1) | 14.4 | 10.1 | 7.2 |
| Polymer (a2) | - | 4.3 | 7.2 |

(continued)

|  | Formulation Example 10 | Formulation Example 11 | Formulation Example 12 |
|---|---|---|---|
| Hydrophobic HPMC | 0.9 | 0.9 | 0.9 |
| DID | 5 | 5 | 5 |
| PS65 | 2 | 2 | 2 |
| Purified water | 77.7 | 77.7 | 77.7 |
| Total amount | 100 | 100 | 100 |
| Weight ratio of (a1) to (a2) | 10:0 | 7:3 | 5:5 |
| Evaluation result of friction resistance | | | |
| Average formulation residual ratio (%) | 95.4 | 99.7 | 99.7 |
| Relative standard deviation (%) | 1.9 | 0.7 | 0.5 |

As shown in Table 5, the formulations containing the polymers (a1) and (a2) at 7 : 3 (Formulation Example 11) and 5 : 5 (Formulation Example 12) showed higher friction resistance than the formulation containing the polymer (a1) alone (Formulation Example 10). From this, it was confirmed that the compositions containing the polymers (a1) and (a2) were excellent in washed-off property, while the compositions showed high resistance to friction, and thus were difficult to remove from the skin.

[Example 4]

A formulation in which the amount of a plasticizer (DID) was changed was prepared, and the washed-off property and the friction resistance were evaluated using the method for evaluating washed-off property described in Example 2 and the method for evaluating friction resistance described in Example 3. The composition and evaluation results of each formulation are shown in Table 6. "Evaluation impossible" in Table 6 indicates that the appearance of film scum during washing was different from that in Formulation Example 9, and therefore evaluation was difficult.

[Table 6]

| | Formulation Example 13 | Formulation Example 14 | Formulation Example 15 | Formulation Example 16 | Formulation Example 17 | Formulation Example 18 | Formulation Example 19 | Formulation Example 20 | Formulation Example 21 | Formulation Example 22 |
|---|---|---|---|---|---|---|---|---|---|---|
| Polymer (a1) | 10.8 | 10.8 | 10.8 | 10.8 | 10.8 | 10.8 | 10.8 | 10.8 | 10.8 | 10.8 |
| Polymer (a2) | 3.6 | 3.6 | 3.6 | 3.6 | 3.6 | 3.6 | 3.6 | 3.6 | 3.6 | 3.6 |
| Hydrophobic HPMC | 0.9 | 0.9 | 0.9 | 0.9 | 0.9 | 0.9 | 0.9 | 0.9 | 0.9 | 0.9 |
| DID | - | 0.5 | 1 | 3 | 6 | 7 | 8 | 9 | 10 | 20 |
| PS65 | 1 | 1 | 1 | 1 | 1 | 1 | 1 | 1 | 1 | 1 |
| Purified water | 83.7 | 83.2 | 82.7 | 80.7 | 77.7 | 76.7 | 75.7 | 74.7 | 73.7 | 63.7 |
| Total amount | 100 | 100 | 100 | 100 | 100 | 100 | 100 | 100 | 100 | 100 |
| Weight ratio of (a1) to (a2) | 3:1 | 3:1 | 3:1 | 3:1 | 3:1 | 3:1 | 3:1 | 3:1 | 3:1 | 3:1 |
| Friction resistance | | | | | | | | | | |
| Formulation residual ratio (%) | 101.1 | 101.1 | 100.4 | 100.4 | 101.4 | 100.8 | 100.3 | 98.5 | 100.4 | 99.8 |
| Relative standard deviation (%) | 0.3 | 0.4 | 1.3 | 0.3 | 0.3 | 0.7 | 0.3 | 0.9 | 0.0 | 0.4 |
| Score of washed-off property by each panelist | | | | | | | | | | |
| Panelist 1 | Evaluation impossible | Evaluation impossible | Evaluation impossible | Evaluation impossible | 3 | 3 | 3 | 3 | 3 | 3 |
| Panelist 2 | 3 | 3 | 3 | 3 | 3 | 3 | 3 | 3 | 3 | Evaluation impossible |
| Panelist 3 | 3 | 3 | 3 | 3 | 3 | 3 | 2 | 2 | 3 | 3 |
| Panelist 4 | 2 | 2 | 2 | 3 | 3 | 2 | 2 | 2 | 2 | 1 |

As shown in Table 6, the friction resistance was good for all formulations. In addition, all formulations satisfied the required washed-off criteria, and in particular, the washed-off property of Formulation Examples 16 to 21 containing 3 to 10% by weight of the plasticizer was good. It was observed that as the amount of the plasticizer increased, the film formation time tended to decrease (quick-drying properties increased), but in Formulation Example 22 containing 20% by weight of the plasticizer, a film was immediately formed when applied to the skin, so that it was difficult to form a uniform film.

[Example 5]

Formulations with different types of plasticizers were prepared, and the washed-off property and the friction resistance were evaluated in the same manner as in Example 4. The composition and evaluation results of each formulation are shown in Table 7.

[Table 7]

| | | Formulation Example 23 | Formulation Example 24 | Formulation Example 25 | Formulation Example 26 | Formulation Example 27 | Formulation Example 28 | Formulation Example 29 |
|---|---|---|---|---|---|---|---|---|
| Polymer (a1) | | 10.8 | 10.8 | 10.8 | 10.8 | 10.8 | 10.8 | 10.8 |
| Polymer (a2) | | 3.6 | 3.6 | 3.6 | 3.6 | 3.6 | 3.6 | 3.6 |
| Hydrophobic HPMC | | 0.9 | 0.9 | 0.9 | 0.9 | 0.9 | 0.9 | 0.9 |
| Plasticizer | Triethyl citrate | 5 | - | - | - | - | - | - |
| | Triacetin | - | 5 | - | - | - | - | - |
| | Diethyl sebacate | | | 5 | | | | |
| | Diisopropyl sebacate | - | - | - | 5 | - | - | - |
| | Medium-chain triglyceride | - | - | - | - | 5 | - | - |
| | Ethylene glycol | - | - | - | - | - | 5 | - |
| | salicylate | | | | | | | |
| | Phenoxyetha nol | - | - | - | - | - | - | 5 |
| PS65 | | 1 | 1 | 1 | 1 | 1 | 1 | 1 |
| Purified water | | 78.7 | 78.7 | 78.7 | 78.7 | 78.7 | 78.7 | 78.7 |
| Total amount | | 100 | 100 | 100 | 100 | 100 | 100 | 100 |
| Weight ratio of (a1) to (a2) | | 3:1 | 3:1 | 3:1 | 3:1 | 3:1 | 3:1 | 3:1 |
| Friction resistance | | | | | | | | |
| Formulation residual ratio (%) | | 80.5 | 75.1 | 97.8 | 100.3 | 97.1 | 91.0 | 99.7 |
| Relative standard deviation (%) | | 11.1 | 9.6 | 1.6 | 3.9 | 4.9 | 5.7 | 0.3 |
| Score of washed-off property by each panelist | | | | | | | | |
| Panelist 1 | | 3 | 3 | 3 | 3 | 3 | 3 | 3 |
| Panelist 2 | | 1 | 2 | 3 | 3 | 4 | 3 | 3 |
| Panelist 3 | | 3 | 4 | 3 | 3 | 4 | 3 | 4 |

14

(continued)

| Score of washed-off property by each panelist | | | | | | | |
|---|---|---|---|---|---|---|---|
| Panelist 4 | 3 | 3 | 3 | 4 | 3 | 4 | 4 |

As shown in Table 7, when triethyl citrate, triacetin or ethylene glycol salicylate was used as the plasticizer, the friction resistance tended to decrease. When diethyl sebacate, diisopropyl sebacate, medium-chain triglyceride or phenoxyethanol was used as the plasticizer, the friction resistance and the washed-off property were good.

[Example 6]

Formulations in which the amount of surfactant (PS65) was changed were prepared, and the washed-off property and the friction resistance were evaluated in the same manner as in Example 4. The composition and evaluation results of each formulation are shown in Table 8.

[Table 8]

| | Formulation Example 30 | Formulation Example 31 | Formulation Example 32 | Formulation Example 33 | Formulation Example 34 | Formulation Example 35 | Formulation Example 36 |
|---|---|---|---|---|---|---|---|
| Polymer (a1) | 10.8 | 10.8 | 10.8 | 10.8 | 10.8 | 10.8 | 10.8 |
| Polymer (a2) | 3.6 | 3.6 | 3.6 | 3.6 | 3.6 | 3.6 | 3.6 |
| Hydrophobic HPMC | 0.9 | 0.9 | 0.9 | 0.9 | 0.9 | 0.9 | 0.9 |
| DID | 5 | 5 | 5 | 5 | 5 | 5 | 5 |
| PS65 | - | 0.5 | 1 | 2 | 3 | 4 | 5 |
| Purified water | 79.7 | 79.2 | 78.7 | 77.7 | 76.7 | 75.7 | 74.7 |
| Total amount | 100 | 100 | 100 | 100 | 100 | 100 | 100 |
| Weight ratio of (a1) to (a2) | 3:1 | 3:1 | 3:1 | 3:1 | 3:1 | 3:1 | 3:1 |
| Friction resistance | | | | | | | |
| Formulation residual ratio (%) | 99.8 | - | 99.6 | - | 98.7 | 99.4 | 96.3 |
| Relative standard deviation (%) | 0.7 | - | 0.7 | - | 0.7 | 0.7 | 1.2 |
| Score of washed-off property by each panelist | | | | | | | |
| Panelist 1 | 2 | 1 | 3 | 3 | 3 | 3 | 3 |
| Panelist 2 | 1 | 1 | 3 | 3 | 3 | 3 | 3 |
| Panelist 3 | 1 | 2 | 3 | 3 | 3 | 4 | 4 |
| Panelist 4 | 1 | 2 | 3 | 2 | 4 | 4 | 4 |

As shown in Table 8, the washed-off property of Formulation Example 30 free of surfactants was not as high as that of Formulation Example 9, but it could be confirmed that the required washed-off criteria were satisfied. Formulation Examples 32 to 36 containing 1% by weight or more of a surfactant showed good washed-off property.

[Example 7]

Formulations with different types of surfactants were prepared, and the washed-off property and the friction resistance were evaluated in the same manner as in Example 4. The composition and evaluation results of each formulation are shown in Table 9 and Table 10.

[Table 9]

| | | Formulation Example 37 | Formulation Example 38 | Formulation Example 39 | Formulation Example 40 | Formulation Example 41 | Formulation Example 42 | Formulation Example 43 |
|---|---|---|---|---|---|---|---|---|
| Polymer (a1) | | 10.8 | 10.8 | 10.8 | 10.8 | 10.8 | 10.8 | 10.8 |
| Polymer (a2) | | 3.6 | 3.6 | 3.6 | 3.6 | 3.6 | 3.6 | 3.6 |
| Hydrophobic HPMC | | 0.9 | 0.9 | 0.9 | 0.9 | 0.9 | 0.9 | 0.9 |
| DID | | 5 | 5 | 5 | 5 | 5 | 5 | 5 |
| Noni onic surfa ctant | NIKKOL BL-4.2 | 1 | - | - | - | - | - | - |
| | NIKKOL TL-10 | - | 1 | - | - | - | - | - |
| | NIKKOL MYO-10V | - | - | 1 | - | - | - | - |
| | NIKKOL Decaglyn 1-L | - | - | - | 1 | - | - | - |
| | NIKKOL DGMO-90V | - | - | - | - | 1 | - | - |
| | NIKKOL TMGS-5V | - | - | - | - | - | 1 | - |
| | NIKKOL TMGS-15V | - | - | - | - | - | - | 1 |
| Purified water | | 78.7 | 78.7 | 78.7 | 78.7 | 78.7 | 78.7 | 78.7 |
| Total amount | 100 | 100 | 100 | 100 | 100 | 100 | 100 | |
| Weight ratio of (a1) to (a2) | 3:1 | 3:1 | 3:1 | 3:1 | 3:1 | 3:1 | 3:1 | |
| Friction resistance | | | | | | | | |
| Formulation residual ratio (%) | 100.5 | 100.5 | 100.0 | 100.0 | 99.8 | 97.8 | 100.3 | |
| Relative standard deviation (%) | 0.1 | 0.9 | 0.0 | 0.6 | 0.3 | 0.9 | 0.3 | |

EP 4 194 018 A1

19

| Score of washed-off property by each panelist | | | | | | | |
|---|---|---|---|---|---|---|---|
| Panelist 1 | 3 | 3 | 3 | 3 | 3 | 3 | 3 |
| Panelist 2 | 3 | 3 | 3 | 3 | 3 | 3 | 3 |
| Panelist 3 | 3 | 2 | 3 | 3 | 2 | 3 | 3 |
| Panelist 4 | 2 | 3 | 3 | 2 | 3 | 3 | 3 |

[Table 10]

| | | Formulation Example 44 | Formulation Example 45 | Formulation Example 46 | Formulation Example 47 |
|---|---|---|---|---|---|
| Polymer (a1) | | 10.8 | 10.8 | 10.8 | 10.8 |
| Polymer (a2) | | 3.6 | 3.6 | 3.6 | 3.6 |
| Hydrophobic HPMC | | 0.9 | 0.9 | 0.9 | 0.9 |
| DID | | 5 | 5 | 5 | 5 |
| Anionic surfactant | NIKKOL Sarcosinate LN | 1 | - | - | - |
| | NONSOUL MK-1 | - | 1 | - | - |
| Amphoteric surfactant | NIKKOL AM-301 | - | - | 1 | - |
| | NIKKOL AM-3130N | - | - | - | 1 |
| Purified water | | 78.7 | 78.7 | 78.7 | 78.7 |
| Total amount | | 100 | 100 | 100 | 100 |
| Weight ratio of (a1) to (a2) | | 3:1 | 3:1 | 3:1 | 3:1 |
| Friction resistance | | | | | |
| Formulation residual ratio (%) | | 100.9 | 99.0 | 98.1 | 99.8 |
| Relative standard deviation (%) | | 0.3 | 0.1 | 1.4 | 0.4 |
| Score of washed-off property by each panelist | | | | | |
| Panelist 1 | | Evaluation impossible | Evaluation impossible | Evaluation impossible | Evaluation impossible |
| Panelist 2 | | Evaluation impossible | Evaluation impossible | Evaluation impossible | Evaluation impossible |
| Panelist 3 | | 3 | 3 | 3 | 3 |
| Panelist 4 | 2 | 2 | 3 | 3 | |

As shown in Table 9, Formulation Examples 37 to 43 containing a nonionic surfactant showed good washed-off property and friction resistance.

As shown in Table 10, the friction resistance was also good when an anionic surfactant or an amphoteric surfactant was used. As for the washed-off property, as shown as "Evaluation impossible" in Table 10, when an anionic surfactant was used, the film was dissolved off during washing, so that it was difficult to determine whether the film had been washed away from the skin. Even when an amphoteric surfactant was used, the panelists 1 and 2 determined that evaluation was impossible, but further, as a result of performing the test regarding the washed-off property (the residue on the skin) shown in Table 1 on 3 panelists, in Formulation Example 46 and Formulation Example 47, 2 panelists had a score of 4 (no residue) and 1 panelist had a score of 3 (30% or less remains), and even when an amphoteric surfactant was used, a formulation having no problem in washed-off property could be prepared.

From the results of Example 7, it was found that when a nonionic surfactant or an amphoteric surfactant is used as a surfactant, it is easy to visually confirm film removal.

[Example 8]

Formulations in which the amounts of the polymers (a1) and (a2) were changed were prepared, and the washed-off property and the friction resistance were evaluated in the same manner as in Example 4. The composition and evaluation results of each formulation are shown in Table 11.

[Table 11]

| | Formulation Example 48 | Formulation Example 49 | Formulation Example 50 | Formulation Example 51 | Formulation Example 52 | Formulation Example 53 | Formulation Example 54 | Formulation Example 55 | Formulation Example 56 | Formulation Example 57 | Formulation Example 58 |
|---|---|---|---|---|---|---|---|---|---|---|---|
| Polymer (a1) | 0.9 | 1.8 | 3.6 | 6.3 | 7.2 | 10.8 | 11.7 | 12.6 | 13.5 | 14.4 | 18.0 |
| Polymer (a2) | 0.3 | 0.6 | 1.2 | 2.1 | 2.4 | 3.6 | 3.9 | 4.2 | 4.5 | 4.8 | 6.0 |
| Hydrophobic HPMC | 0.9 | 0.9 | 0.9 | 0.9 | 0.9 | 0.9 | 0.9 | 0.9 | 0.9 | 0.9 | 0.9 |
| DID | 0.42 | 0.83 | 1.67 | 2.92 | 3.33 | 5.00 | 5.42 | 5.83 | 6.25 | 6.67 | 8.33 |
| PS65 | 1 | 1 | 1 | 1 | 1 | 1 | 1 | 1 | 1 | 1 | 1 |
| Purified water | 96.48 | 94.87 | 91.63 | 86.78 | 85.17 | 78.70 | 77.08 | 75.47 | 73.85 | 72.23 | 65.77 |
| Total amount | 100 | 100 | 100 | 100 | 100 | 100 | 100 | 100 | 100 | 100 | 100 |
| Weight ratio of (a1) to (a2) | 3:1 | 3:1 | 3:1 | 3:1 | 3:1 | 3:1 | 3:1 | 3:1 | 3:1 | 3:1 | 3:1 |
| Friction resistance | | | | | | | | | | | |
| Formulation residual ratio (%) | 83.0 | 101.1 | 102.8 | 101.2 | 100.7 | 99.6 | 100.3 | 100.7 | 99.9 | 99.7 | 101.6 |
| Relative standard deviation (%) | 35.9 | 6.5 | 2.3 | 1.1 | 3.5 | 0.7 | 0.7 | 0.3 | 0.8 | 1.0 | 0.8 |
| Score of washed-off property by each panelist | | | | | | | | | | | |
| Panelist 1 | 3 | 3 | 3 | 3 | 3 | 3 | 3 | 3 | 3 | 3 | 3 |
| Panelist 2 | 4 | 4 | 4 | 3 | 3 | 3 | 3 | 3 | 3 | 3 | 2 |
| Panelist 3 | 3 | 3 | 3 | 4 | 3 | 3 | 3 | 3 | 3 | 3 | 3 |
| Panelist 4 | 3 | 3 | 3 | 3 | 4 | 3 | 3 | 3 | 3 | 4 | 4 |

As shown in Table 9, Formulation Example 48 in which the total amount of the polymers (a1) and (a2) was 1.2% by weight had low friction resistance as compared with other formulations. On the other hand, Formulation Examples 49 to 58 in which the total amount of the polymers (a1) and (a2) was 2.4% by weight to 24% by weight showed good washed-off property and friction resistance.

**Claims**

1.  A film-forming skin composition comprising the following two polymers:

    (a1) an ethyl acrylate-methyl methacrylate-trimethylammonium ethyl methacrylate chloride copolymer; and
    (a2) an ethyl acrylate-methyl methacrylate copolymer.

2.  The composition according to claim 1, comprising propranolol or a pharmaceutically acceptable salt thereof as a medicinal ingredient.

3.  The composition according to claim 2, which is used for treatment of hemangioma.

4.  The composition according to claim 2 or 3, which is used for treatment of hemangioma in infants.

5.  The composition according to any one of claims 1 to 4, wherein a weight ratio of the polymer (a1) to the polymer (a2) is in a range of 8 : 2 to 2 : 8.

6.  The composition according to any one of claims 1 to 5, further comprising 36% by weight or more of water based on the total amount of the composition (provided that a propellant is excluded when the composition is an aerosol).

7.  The composition according to any one of claims 1 to 6, further comprising a surfactant.

8.  The composition according to claim 7, wherein the surfactant is a nonionic surfactant and/or an amphoteric surfactant.

9.  The composition according to any one of claims 1 to 8, further comprising one or more plasticizers selected from the group consisting of an ester compound that is liquid at ordinary temperature, an aromatic alcohol that is liquid at ordinary temperature, a medium-polar solid ester compound, and a terpene.

10. The composition according to any one of claims 1 to 9, wherein the composition is substantially free of a lower monohydric alcohol.

## INTERNATIONAL SEARCH REPORT

International application No.

**PCT/JP2021/028942**

### A. CLASSIFICATION OF SUBJECT MATTER

*A61L 26/00*(2006.01)i; *A61K 47/32*(2006.01)i
FI: A61L26/00; A61K47/32

According to International Patent Classification (IPC) or to both national classification and IPC

### B. FIELDS SEARCHED

Minimum documentation searched (classification system followed by classification symbols)

A61L26/00; A61K47/32

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| A | JP 5-32535 A (ZENYAKU KOGYO KABUSHIKI KAISHA) 09 February 1993 (1993-02-09)<br>claim 1, examples 1-10 | 1-10 |
| A | JP 2008-100966 A (KYOWA LTD.) 01 May 2008 (2008-05-01)<br>claim 1, examples 1-12 | 1-10 |
| A | JP 10-226639 A (SHISEIDO CO., LTD.) 25 August 1998 (1998-08-25)<br>claims 1-3, tables 1-4 | 1-10 |
| A | JP 3-7238 A (YAMANOUCHI PHARMACEUTICAL CO., LTD.) 14 January 1991 (1991-01-14)<br>claim 1, example 1 | 1-10 |
| A | JP 7-277975 A (POLA CHEMICAL INDUSTRIES, INC.) 24 October 1995 (1995-10-24)<br>claim 1, examples 1-12 | 1-10 |
| A | JP 2011-126796 A (KRACIE HOME PRODUCTS, LTD.) 30 June 2011 (2011-06-30)<br>claim 1, examples 1-9 | 1-10 |

☑ Further documents are listed in the continuation of Box C.   ☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **29 September 2021** | **12 October 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**EP 4 194 018 A1**

**INTERNATIONAL SEARCH REPORT**

| International application No. |
|---|
| **PCT/JP2021/028942** |

**C. DOCUMENTS CONSIDERED TO BE RELEVANT**

| Category* | Citation of document, with indication, where appropriate, of the relevant passages | Relevant to claim No. |
|---|---|---|
| A | JP 2011-126797 A (KRACIE HOME PRODUCTS, LTD.) 30 June 2011 (2011-06-30) claim 1, examples 1-12 | 1-10 |
| A | 高塚 博一ほか, 乳児血管腫に対する院内製剤チモロールゲルの製剤学的評価, 医療薬学, 2017, vol. 43, no. 12, pp. 706-712., ISSN:1882-1499, (TAKATSUKA, Hirokazu et al., "Pharmaceutical Evaluation of the Hospital Grade Preparation of Timolol Gel for Infantile Hemangioma", Iryo Yakugaku) introduction | 1-10 |

Form PCT/ISA/210 (second sheet) (January 2015)

**INTERNATIONAL SEARCH REPORT**
Information on patent family members

International application No.

**PCT/JP2021/028942**

| Patent document cited in search report | | | Publication date (day/month/year) | Patent family member(s) | | | Publication date (day/month/year) |
|---|---|---|---|---|---|---|---|
| JP | 5-32535 | A | 09 February 1993 | (Family: none) | | | |
| JP | 2008-100966 | A | 01 May 2008 | (Family: none) | | | |
| JP | 10-226639 | A | 25 August 1998 | TW | 493990 | B | |
| JP | 3-7238 | A | 14 January 1991 | US | 5028664 | A | |
| | | | | claim 1, example 1 | | | |
| | | | | EP | 386967 | A2 | |
| | | | | DE | 69020758 | C | |
| | | | | NO | 301578 | B | |
| | | | | NZ | 232836 | A | |
| | | | | PT | 93384 | A | |
| | | | | ES | 2077023 | T | |
| | | | | DD | 292374 | A | |
| | | | | DK | 386967 | T3 | |
| | | | | HU | 53813 | A | |
| | | | | IE | 68520 | B | |
| | | | | AT | 124864 | T | |
| | | | | AU | 5122790 | A | |
| | | | | CA | 2011919 | A1 | |
| | | | | GR | 3017645 | T | |
| | | | | KR | 10-1990-0013943 | A | |
| | | | | CN | 1045524 | A | |
| | | | | FI | 901195 | A0 | |
| JP | 7-277975 | A | 24 October 1995 | (Family: none) | | | |
| JP | 2011-126796 | A | 30 June 2011 | (Family: none) | | | |
| JP | 2011-126797 | A | 30 June 2011 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011126796 A
- JP 2011126797 A
- JP H0532535 A

**Non-patent literature cited in the description**

- *Pharmaceutical Bulletin,* 1954, vol. 2 (2), 163-173
- *Chemistry region,* 1957, vol. 11 (10), 719-725
- *Fragrance journal,* 1981, vol. 50, 79-82